# EUROPEAN PATENT SPECIFICATION

(11) **EP 4 347 125 B1**
(45) Date of publication and mention of the grant of the patent: **02.04.2025**
(21) Application number: 22730612.3
(22) Date of filing: 20.05.2022
(51) Int. Cl.: A61B 5/154, B65D 39/04, A61B 5/15, B01L 3/00, B65D 41/04

(54) **HERMETICALLY SEALABLE CONTAINER FOR LIQUIDS**
HERMETISCH VERSIEGELBARER BEHÄLTER FÜR FLÜSSIGKEITEN
CONTENANT HERMÉTIQUEMENT SCELLABLE POUR LIQUIDES

(30) Priority: 26.05.2021 IT 202100013640
(43) Date of publication of application: 10.04.2024
(73) Proprietor: Vacutest Kima S.r.l., 35020 Arzergrande, Padova (IT)
(72) Inventor: CHIARIN, Renzo, 35020 Arzergrande, Padova (IT); CHIARIN, Ulisse, 35020 Arzergrande, Padova (IT)
(74) Representative: Zanettin, Gianluigi
(86) International application number: PCT/IB2022/054732
(87) International publication number: WO 2022/249004

(56) References cited:
- EP-A1- 2 030 688
- CA-C- 1 326 809
- FR-A- 1 335 073
- FR-A1- 2 493 274
- US-A- 4 712 699
- US-A- 5 954 215
- US-A1- 2020 172 298
- US-A1- 2021 039 090

## Description

### FIELD OF APPLICATION

The present invention refers to a hermetically sealable container for liquids.

The hermetically sealable container for liquids according to the invention may be used for the containment of any liquid.

Specifically, the container according to the invention is intended for the containment of liquids for which the risk of conveying the contents from the container to the external environment is to be reduced by means of the sealing cap.

Still more specifically, the container according to the invention is intended for the containment of contaminating and/or hazardous liquids, such as biological samples. In this case, the container may be made, in particular, in the form of a jar or test tube.

### PRIOR ART

A hermetically sealable container for liquids comprises a container body, which delimits the volume of the container, and a cap, which is configured to close the mouth of the container body and make a hermetic seal therewith to prevent the contents from escaping.

Generally, a hermetic seal is achieved by interposing a gasket made of elastomeric material between the container body and the cap.

An alternative solution involves equipping the cap with an appendage made of elastic polymer material, intended to be inserted inside the container body. The appendage is dimensioned to couple by interference with the inner walls of the container and by deforming, thus forming a hermetic seal. This solution is adopted especially for vacuum-sealed tubes.

There are also containers in which the caps do not have appendages made of polymeric material, but in which the cap and container body are both made entirely of plastics material and the hermetic seal is achieved without the use of elastomeric gaskets. These containers are more economical to make. The cap may be coupled to the container by screwing or by simple pressure.

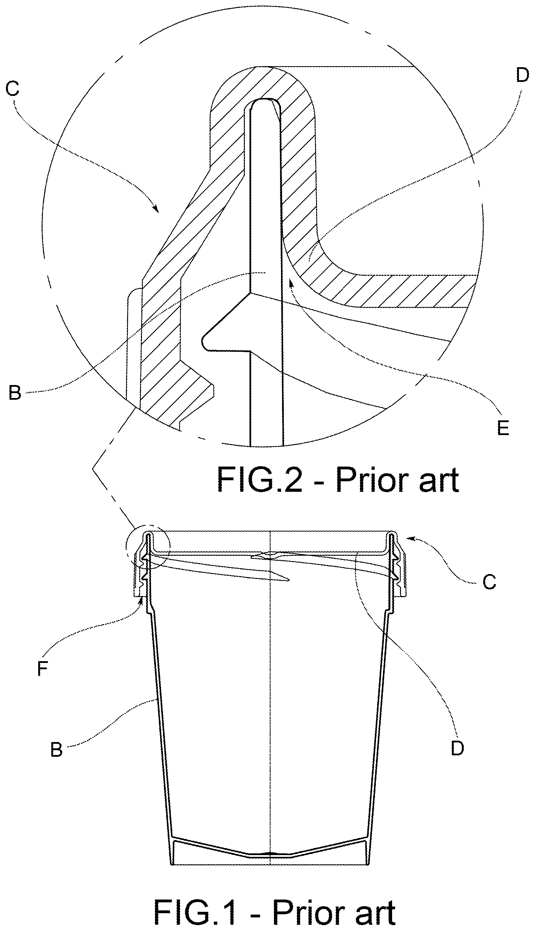

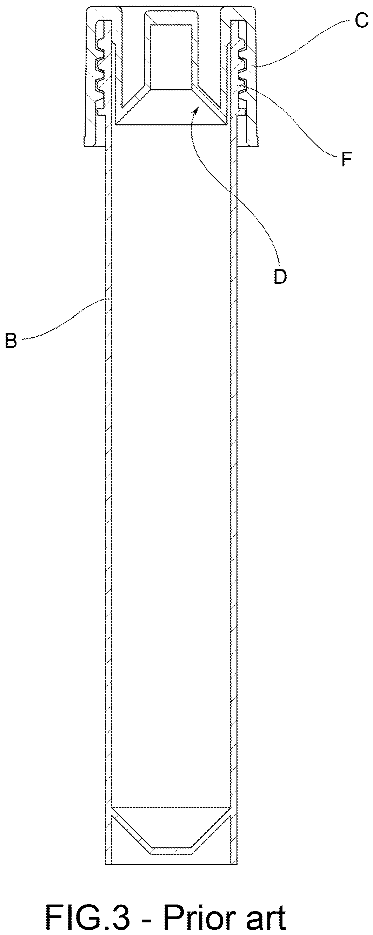

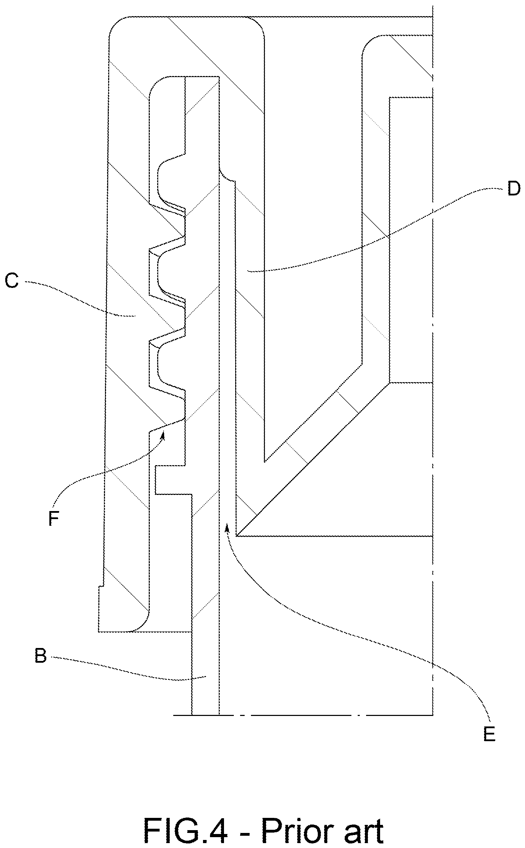

In the containers shown, for example, in Fig. 1 to 4 (jar and swab tube), the cap C is screwed onto the container body B, and for this purpose the container body has a threaded portion F formed on the outer surface near the mouth. The cap C further comprises an inner appendage D intended to fit inside the mouth of the container body. Said inner appendage D is slightly conical so as to allow easy insertion of said appendage into the mouth and is dimensioned so that, past a certain level of screwing of the cap onto the container body, the appendage begins to interfere with the inner walls of the container body. A hermetic seal is achieved by forcing the cap by screwing until it comes to rest on the edge of the mouth.

Because of its conicity, the inner appendage D does not adhere to the inner walls of the container body along its entire length, but is spaced therefrom at least at its head portion. An annular cavity E is then formed between the appendage D and the container body B, which cavity, due to capillary phenomena, becomes an area wherein some of the liquid contents of the container may easily stagnate and accumulate. In fact, it is not uncommon for a container to be shaken or tilted enough during transport or handling to cause the contents to lap against the cap. Upon opening the container, the liquid accumulated in the aforesaid annular cavity E is pulled away by the cap and may accidentally leak into the environment in which the container is handled.

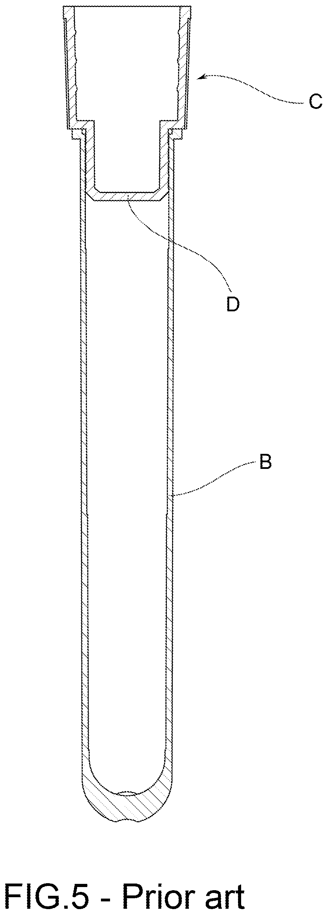

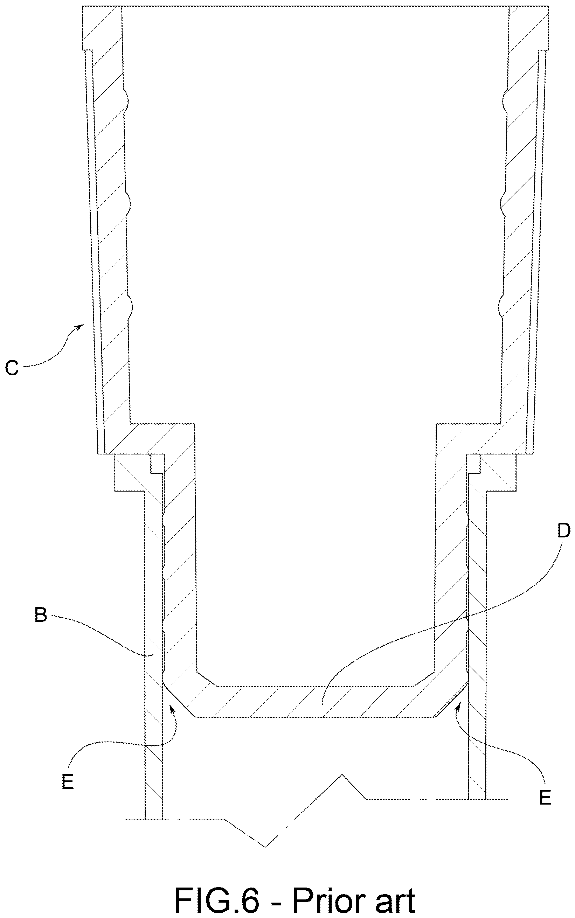

The same issue arises if the plastics cap is configured to engage the container body under pressure, as in the example of the container (test tube) shown in Fig. 5 and 6. Also in this case, in fact, the cap C is equipped with an inner appendage D that is intended to fit inside the container body B and that inevitably forms an annular cavity E because of the necessary conicity with which the head portion of the inner appendage D is endowed.

This issue is particularly acute when containers are intended to contain potentially contaminating and/or hazardous liquids. In the case of biological samples that are potentially contaminated with pathogens, this increases the risk of contamination of the environments and/or operators handling said samples.

Although operators assigned to handle containers containing biological specimens are aware of these risks and are therefore able to put in place procedures to minimize them, a need is felt in the relevant sector for hermetically sealable containers that are configured to inherently reduce the risk of contamination due to the conveyance of the biological specimen to the external environment via the container lid.

Conveying the contents outside the container via the cap may be problematic even if this does not involve biological and/or hazardous liquids. One may consider the case of liquids that may soil or stain.

Thus, the need for hermetically sealable containers that are configured to inherently reduce the risk of contamination due to the conveyance of contents to the external environment via the container cap cuts across many areas of application. Hermetically sealable containers seeking to solve the drawbacks outlined above are disclosed in documents CA1326809C and US5954215A. These documents, however, do not disclose the structural configurations of the cap's coupling appendage and of the inner surface of the container wall, which produce the specific inference configuration between them, as described in the characterizing portion of the appended independent claim, that is responsible for creating three sealing zones between the cap and container body.

To date, however, this need is completely unsatisfied as no hermetically sealable containers for liquids, which are made entirely of plastics and which do not have the drawbacks described above, are available.

### DISCLOSURE OF THE INVENTION

Thus, the main object of the present invention is to eliminate or at least mitigate the drawbacks of the aforesaid prior art by providing a hermetically sealable container for liquids that, while being completely made of plastics, allows for the significant reduction of the risk of contamination by conveying the contents to the external environment via said container cap.

A further object of the present invention is to provide a hermetically sealable container for liquids that ensures a hermetic seal comparable to similar containers of known types.

A further object of the present invention is to provide a hermetically sealable container for liquids which is simple and inexpensive to manufacture.

### BRIEF DESCRIPTION OF THE DRAWINGS

The technical features of the invention, according to the aforesaid objects, may be clearly seen in the contents of the claims below, and its advantages will become more readily apparent in the detailed description that follows, made with reference to the accompanying drawings, which represent one or more purely exemplifying and non-limiting embodiments thereof, wherein:
- Fig. 1 is an orthogonal elevation view in cross section of an example of a hermetically sealable container for liquids of a known type, consisting of a biological specimen jar fitted with a screw-on cap;
- Fig. 2 is an enlarged detail of the container of Fig. 1;
- Fig. 3 is an orthogonal elevation view in cross section of a further example of a known type of a hermetically sealable container for liquids, consisting of a swab tube equipped with a screw-on cap;
- Fig. 4 is an enlarged detail of the container of Fig. 3;
- Fig. 5 is an orthogonal elevation view in cross section of a further example of a known type of a hermetically sealable container for liquids, consisting of a test tube fitted with a pressure cap;
- Fig. 6 is an enlarged detail of the container of Fig. 1;
- Fig. 7 shows an orthogonal elevation view in cross section of a hermetically sealable container for liquids according to a first embodiment of the invention, consisting of a jar for biological samples fitted with a screw-on cap;
- Fig. 8 is an enlarged detail of the container of Fig. 7;
- Fig. 9 is an enlarged detail of the cap of the container in Fig. 8 separated from the container body;
- Fig. 10 is an enlarged detail of the container body of the container of Fig. 8 separated from the cap;
- Fig. 11 is an orthogonal elevation view in cross section of a hermetically sealable container for liquids according to a second embodiment of the invention, comprised of a swab tube fitted with a screw-on cap;
- Fig. 12 is an enlarged detail of the container of Fig. 11;
- Fig. 13 is a further enlarged detail of the container in Fig. 12;
- Fig. 14 is an enlarged detail of the container cap in Fig. 13 separated from the container body;
- Fig. 15 is an enlarged detail of the container body of the container in Fig. 13 separated from the cap;
- Fig. 16 is an orthogonal elevation view in cross section of a hermetically sealable container for liquids according to a third embodiment of the invention, consisting of a test tube fitted with a pressure cap;
- Fig. 17 is an enlarged detail of the container in Fig. 16;
- Fig. 18 is an enlarged detail of the container body of the container of Fig. 17 separated from the cap; and
- Fig. 19 is an enlarged detail of the container cap in Fig. 17 separated from the container body.

### DETAILED DESCRIPTION

The hermetically sealable container for liquids according to the invention has been denoted collectively with 1 in the attached figures.

Herein and in the following description and the claims, reference will be made to the container 1 in the condition of use. Therefore, any references to a lower or upper position or to a horizontal or vertical orientation should be interpreted in this sense.

According to a general embodiment of the invention, the hermetically sealable container 1 for liquids comprises a container body 10, which delimits the inner volume of the container laterally by a tubular container wall 11 and at the bottom by a container bottom 12, from which said tubular container wall 11 extends. The container body 10 has a mouth 13 delimited by a free edge 14 of said tubular wall 11.

An inner surface area 110 and an outer surface area 120 may be identified on the tubular body 11.

The hermetically sealable liquid container 1 further comprises a cap 20, configured to close the mouth 13 of container body 10 and form a hermetic seal therewith.

In turn, the cap 20 comprises a main gripping body 21, which is intended to remain external to the container body 10 and through which the cap is manipulable by a user.

The cap 20 further comprises a coupling appendage 22, which:
- is intended to be inserted inside the container body 10 through the mouth 13,
- extends axially between a base 220 and a head end 221, and
- comprises a lateral coupling surface 23 intended to face the inner surface 110 of said tubular container part 11.

The container body 10 and the cap 20 are made completely of plastics.

Preferably, both the container body 10 and the cap 20 are made by injection molding of plastics material.

Preferably, the container body 10 is made of a plastics material chosen from the group consisting of polypropylene, polyethylene, polystyrene, PET, PVC, polycarbonate, methacrylate, and the copolymers thereof.

Preferably, the cap 20 is made of a plastics material chosen from the group consisting of polypropylene, polyethylene, polystyrene, PET, PVC, polycarbonate, methacrylate, and the copolymers thereof.

According to a first aspect of the invention, the inner surface 110 of said tubular container wall 11 comprises sequentially from the free edge 14 toward the bottom 12:
- a conical guide surface 111, converging toward the bottom 12;
- an intermediate surface 112, connecting said conical guide surface 111 to a shoulder 113;
- an end surface 114 connecting said shoulder 113 to the container bottom 12.

Functionally, the conical guide surface 111 is suitable to facilitate the insertion of the coupling appendage 22 of the cap 20 inside the container body 10 through the mouth 13. For this purpose, the conical guide surface 111 has a minimum diameter greater than the maximum diameter of the coupling appendage 22, with the exclusion of specific portions of the coupling appendage 22, which, as will be taken up in the following, are specifically dimensioned to engage in an interference relationship on the conical guide surface 111.

Advantageously, the aforesaid conical guide surface 111 has a conicity between 1/1 and 1/50.

The conicity C is a dimensionless quantity that expresses the ratio between the difference of the diameters D and d of two cross sections of a cone and the axial distance L between them, expressed by the relationship C = (D-d)/L. For example, a conicity C equal to 1:20 means that, for an axial distance of 20 millimeters between two cross sections, the major diameter D and minor diameter d will differ by 1 millimeter.

Functionally, the intermediate surface 112 is suitable to define a connection surface between the conical guide surface 111 and the shoulder 113.

The aforesaid intermediate surface 112 may be conical converging toward the bottom 12 or cylindrical.

Preferably, if the intermediate surface 112 is conical, it has a conicity lower than the conicity of the conical guide surface 111.

The aforesaid intermediate surface 112 defines a first annular prominence 115 in the vicinity of said shoulder 113. Functionally, said first annular prominence 115 is suitable to define a localized bottleneck section of the tubular container wall 11 at the inner surface 110.

According to a second aspect of the invention, the coupling appendage 22 of said cap 20 has an axial extension such that the coupling appendage 22 may be inserted within the container body 11 until it reaches said shoulder 113 with the head end 221. In other words, the coupling appendage 20 has an axial development (distance between the base 220 and the head end 221) at least equal to the distance between the free edge 14 and the shoulder 113.

The coupling appendage 22 of said cap 20 also has a radial extension (in a plane cross-sectional to the axis) at the head end 221 such that, when it reaches the shoulder 113, the head end 221 abuts against said shoulder 113, so as to create a first sealing zone T1 between the cap (20) and the container body 10.

According to a third aspect of the invention, the head end portion 221 of the coupling appendage 22 is radially dimensioned such that when the head end 221 abuts against the shoulder 113, the head end portion 221 interferes with the aforesaid first annular prominence 115, creating a second sealing zone T2 between the cap 20 and the container body 10.

According to a fourth aspect of the invention, the coupling appendage 22 of said cap (20) comprises a second annular prominence 222 which protrudes from said lateral coupling surface 23 in the vicinity of the base 220 of said coupling appendage 22 and is positioned axially such that, when the head end 221 abuts against said shoulder 113, the second annular prominence 222 interferes with the conical guide surface 111, creating a third sealing zone T3 between the cap 20 and the container body 10.

Due to the invention, the coupling appendage 22 adheres to the inner surface 110 of the tubular wall 11 of the container body 10 at least at its own head end 221. More specifically, by creating an initial sealing zone T1 at the head end 221, the seepage of the container contents between the coupling appendage 22 and the tubular wall 11 is prevented. Thus, an open annular cavity is not formed between the coupling appendage 22 and the container body 10 on the inside of said container. All this prevents the contents of the container from seeping between the coupling appendage and the container body and stagnating in said position. Therefore, when the container is opened, as there is no liquid accumulated between the appendage and the container body, the risk of the contents being conveyed outside the container via said cap is significantly reduced.

This result is achieved without making the coupling appendage 22 of elastomeric material or even endowing this appendage with seals made of elastomeric material.

The insertion of the coupling appendage inside the container body is facilitated by the conical guide surface 111 formed near the free edge 14 of the container body. This allows for easy insertion inside the container body without making the head end portion of the coupling appendage conical. The absence of conicity on the head end portion 221 of the coupling appendage synergistically helps to avoid the creation of annular cavities (i.e., areas of potential liquid stagnation) between the coupling appendage and tubular wall.

The seepage of liquid between the coupling appendage and the tubular wall is also counteracted by the second sealing zone T2 made between the tubular wall 11 and the head end portion 221 near the shoulder 113.

Preferably, precisely to enhance the effectiveness of the aforesaid second sealing zone T2, the intermediate surface 112 defines the first annular prominence 115 against said shoulder 113. In other words, the first annular prominence 115 connects directly with the shoulder 113. Thus, the second sealing zone T2 is seamlessly connected to the first sealing zone T1.

Functionally, the seal generated at the first sealing zone T1 results from the axial compression of the coupling appendage 22 against the tubular wall 11 at the shoulder 113. Conversely, the seal generated at the second sealing zone T2 results from the radial compression of the coupling appendage 22 against the tubular wall 11 at the first annular prominence 115.

The third sealing zone T3 between the coupling appendage 22 and the tubular wall 11, due to the interference between the conical guide surface 111 and second annular prominence 222, has a dual function:
- improving the hermetic seal of the cap 22 on the container body; and
- keeping the coupling appendage 22 axially centered within the tubular wall 11 so as to avoid radial movements of the cap relative to the container body; said movements could in fact negatively interfere with the sealing effectiveness of the first and second sealing zones.

From the foregoing, it clearly appears that the hermetically sealable container 1 for liquids according to the invention, while being completely made of plastics, allows for a significant reduction in the risk of contamination by conveying the contents to the external environment via the container cap.

Further, the container 1 according to the invention provides a hermetic seal comparable to similar containers of a known type due to the presence of as many as three sealing zones T1, T2 and T3.

Advantageously, the coupling surface 23 of the coupling appendage 22 is substantially cylindrical, except for the portion on which the second annular prominence 222 is formed.

The term "substantially cylindrical" is also intended to include cases in which the coupling appendage has a coupling surface 23 endowed with a slight or very slight conicity, imposed by molding production requirements.

In other words, the coupling appendage 22 may be comprised of a cylindrical or slightly conical body for the aforesaid reasons. Preferably, the maximum conicity of the coupling appendage 22 is 1/1.

Preferably, the head end 222 is counter-shaped with respect to the shoulder 113 in order to improve the fit between the head end and shoulder and thus ensure a better fit.

Advantageously, the cap 20 may be of the type to be engaged by screwing onto the container body 10 at the main gripping body 21.

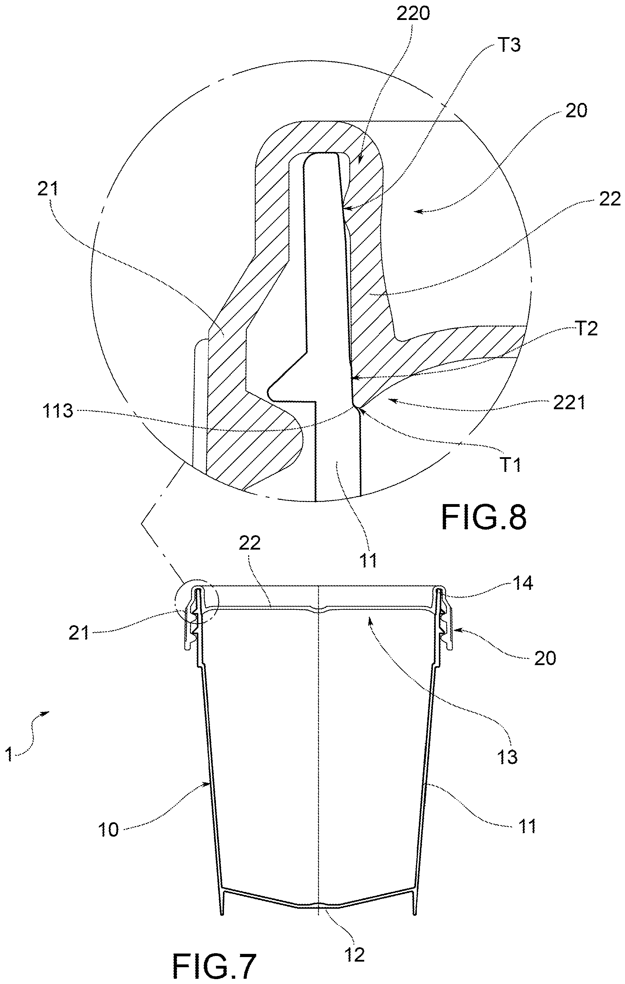

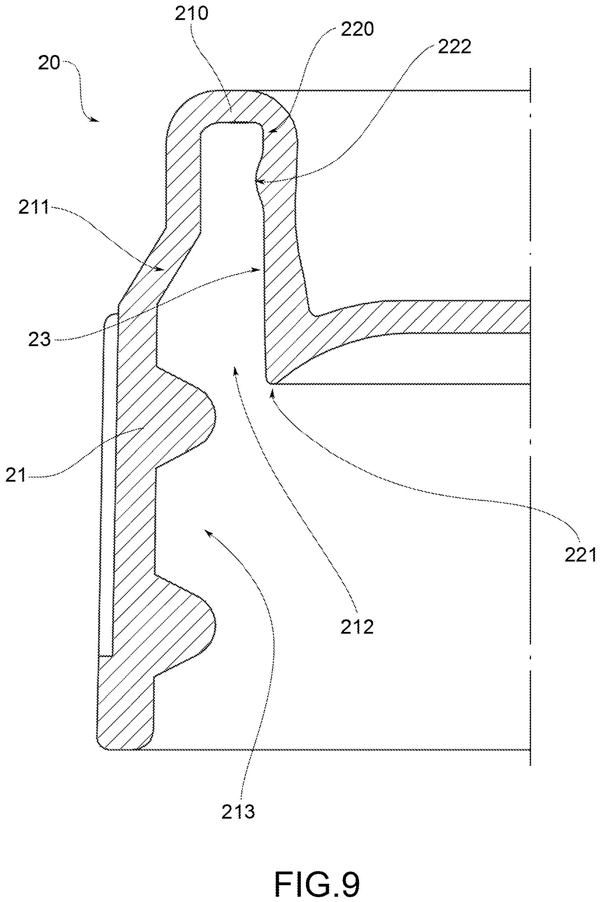

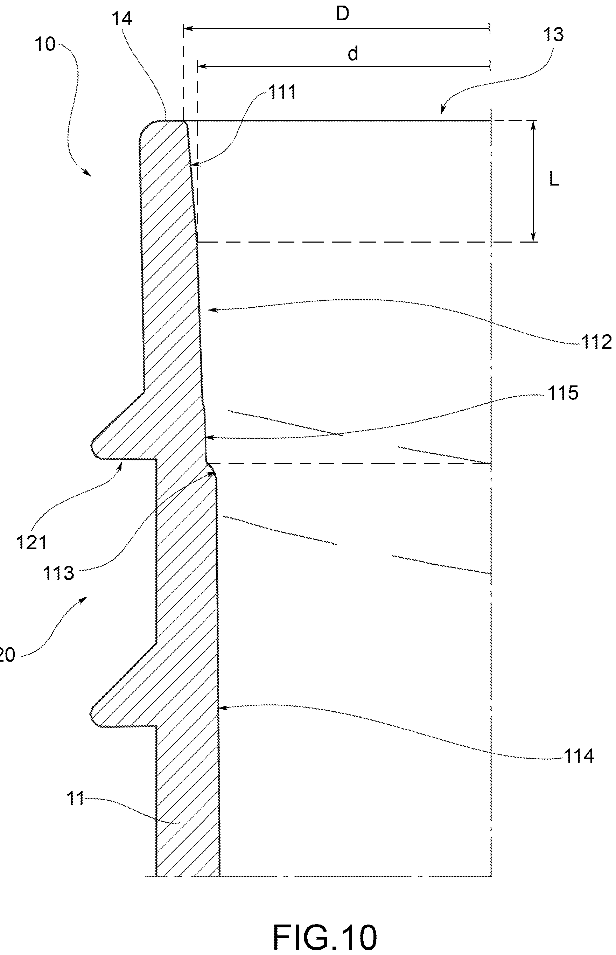

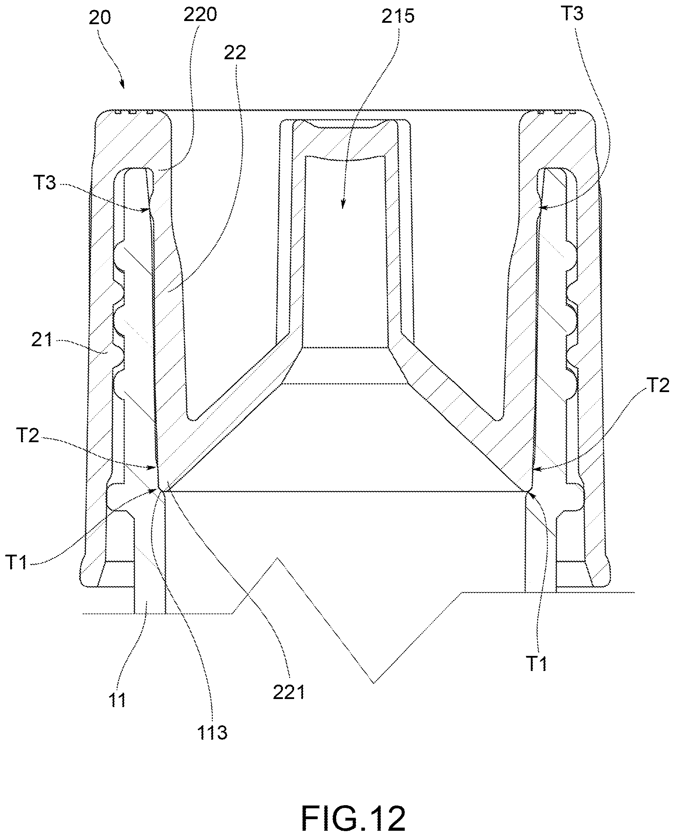

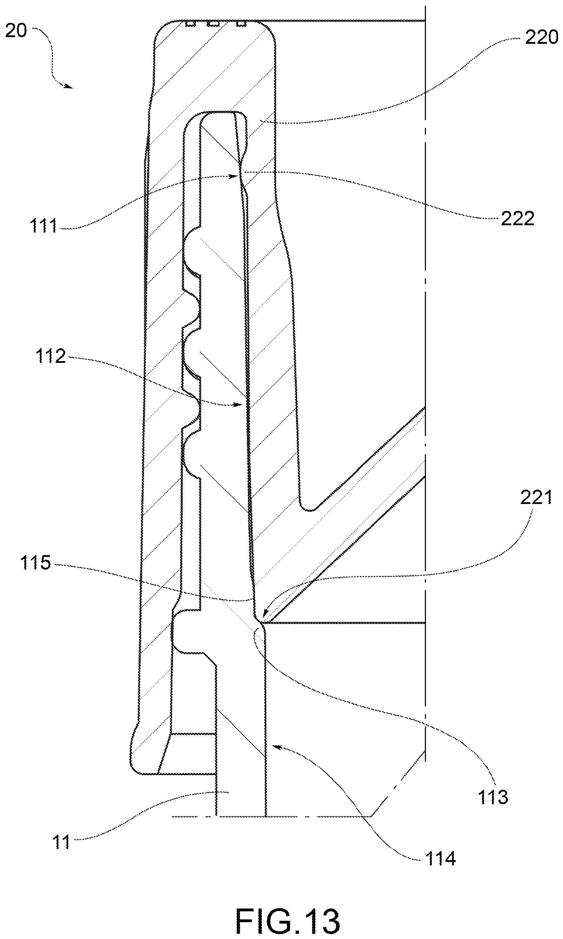

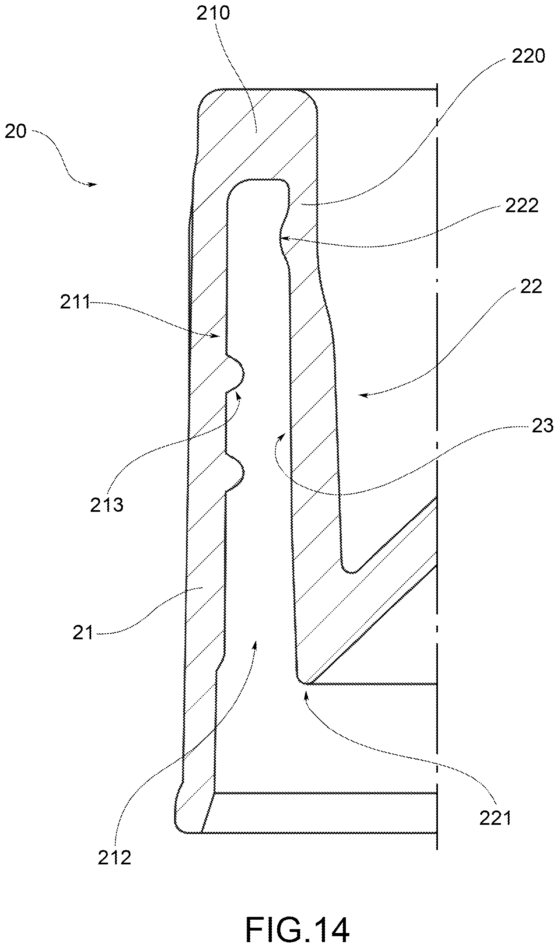

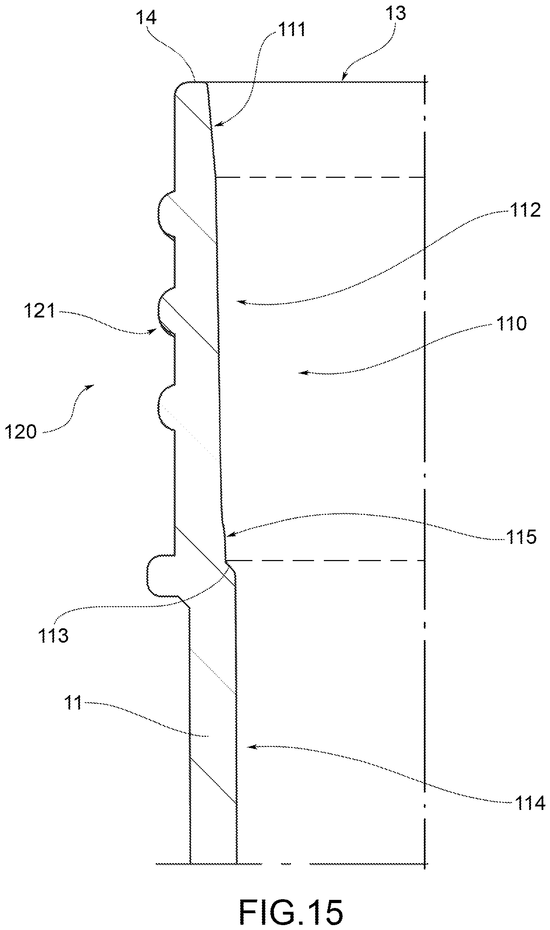

Specifically, as shown in the two embodiments of Fig. 7 to 10 and 11 to 15 respectively, the main gripping body 21 is comprised of a cup-shaped body having a bottom 210 and a side wall 211. The coupling appendage 22 extends coaxially within the cup-shaped body from the bottom 210 such that an annular cavity 212 intended to partially accommodate therein a portion of said container body 10 is defined between the side wall 211 of said cup-shaped body and the coupling appendage. The side wall 211 comprises a threaded surface 213 that faces the inside of said cup-shaped body and is intended to engage in screwing with a counter-threaded surface 121 formed on the outer surface 120 of said container body 10.

Operatively, in such a case, the insertion of the coupling appendage 22 inside the container body 10 is guided and forced by screwing the cap onto the container body.

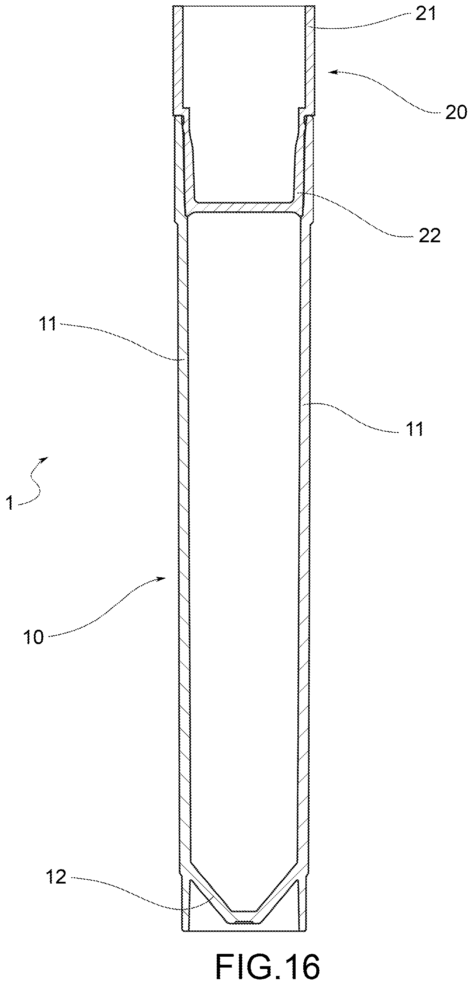

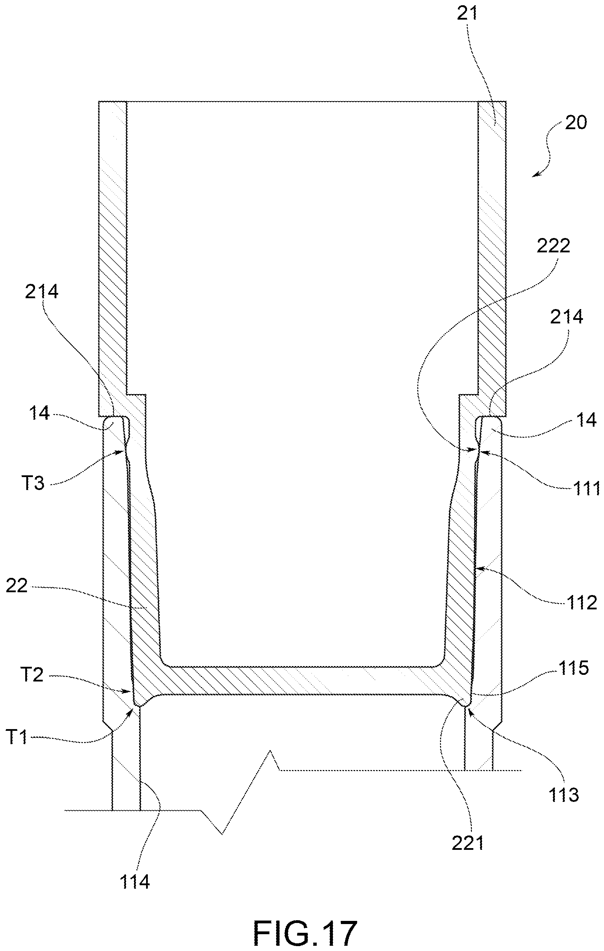

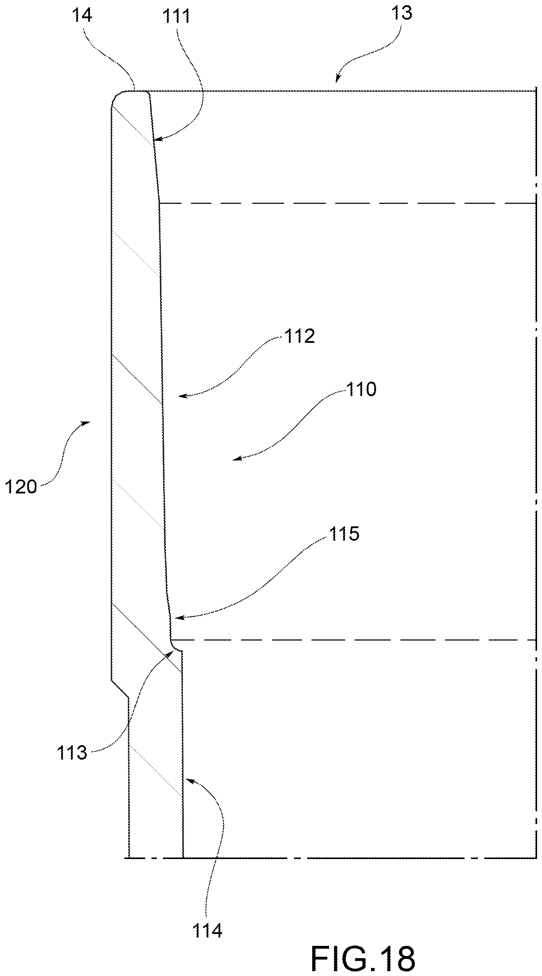

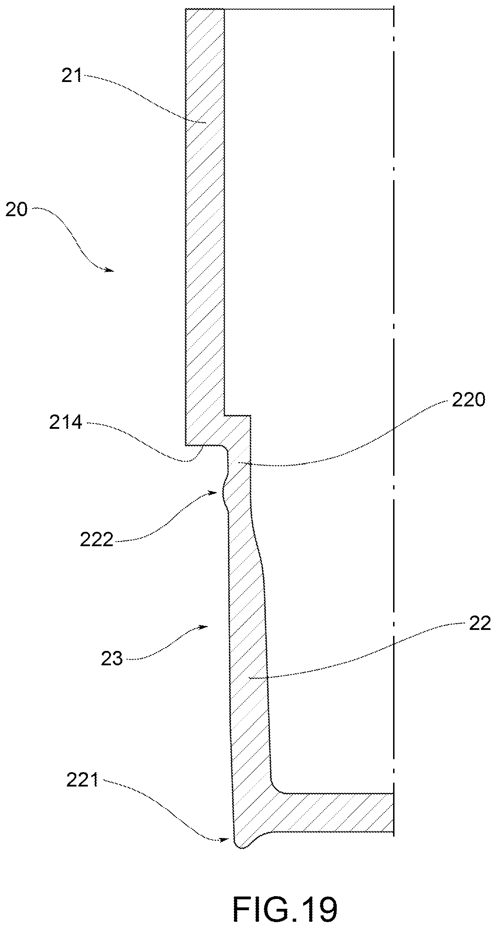

Alternatively, as shown in the embodiment of Fig. 16 to 19, the cap 20 may also not be engageable by screwing onto the container body 10, but rather be a cap that is simply insertable by pressure, possibly by applying a relative rotational motion between the cap and the container body.

Specifically, as shown in Fig. 16 to 19, the aforesaid main gripping body 21 defines, at the base 220 of the coupling appendage 22, an abutment ring 214 for the free edge 14 of said container body 10. The main gripping body 21 is configured to engage said container body 10 only at said abutment ring 214.

The hermetically sealable container 1 for liquids according to the invention may be used for the containment of any liquid.

Specifically, the container according to the invention is intended for the containment of liquids for which the risk of conveying the contents from the container to the external environment is to be reduced by means of the sealing cap.

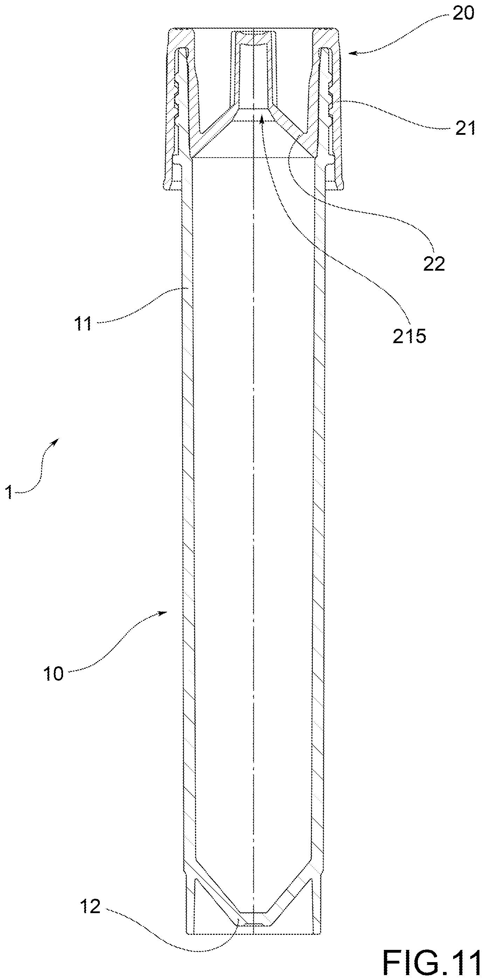

Still more specifically, the container according to the invention is intended for the containment of contaminating and/or hazardous liquids, such as biological samples. In this case, advantageously, the container may be made, in particular, in the form of a jar (as shown in Fig. 7 to 10) or a test tube (as shown in Fig. 11 to 15 and Fig. 16 to 19).

In particular, the container 1 may be made in the form of a swab tube, in which, as shown in Fig. 11 to 15, the coupling appendage 22 may be fitted with a seat 215, intended to be engaged by the support rod of a swab (not shown in the figures).

The invention allows numerous advantages to be obtained which have been explained throughout the description.

The hermetically sealable container 1 for liquids according to the invention, while being made entirely of plastics, significantly reduces the risk of contamination by conveying the contents to the external environment via the container cap.

The hermetically sealable container 1 for liquids according to the invention further provides a hermetic seal comparable to similar containers of known types.

Finally, the hermetically sealable container 1 for liquids according to the invention is simple and inexpensive to make, as it may be made in particular by the injection molding of plastics material.

The invention thus conceived therefore achieves its intended objects.

## Claims

1. Hermetically sealable container (1) for liquids comprising:
- a container body (10), delimiting the internal volume of the container laterally by means of a tubular container wall (11) and underneath by a container bottom (12), from which said tubular container wall (11) extends, said container body being provided with a mouth (13) delimited by a free edge (14) of said tubular wall (11); and
- a cap (20), configured to close the mouth (13) of the container body (10) and make a hermetic seal therewith,
wherein said cap (20) comprises a main gripping body (21), which is intended to remain outside said container body (10), and a coupling appendage (22), which is intended to be inserted inside said container body (10) through said mouth (13), extends axially between a base (220) and a head end (221), and comprises a lateral coupling surface (23) intended to face an inner surface (110) of said tubular container wall (11),
**characterized in that** the inner surface (110) of said tubular container wall (11) sequentially comprises starting from the free edge (14) towards the bottom (12):
- a conical guide surface (111) converging towards the bottom (12); - an intermediate surface (112) connecting said conical guide surface (111) to a shoulder (113); - an end surface (114) connecting said shoulder (113) to the container bottom (12), wherein said intermediate surface (112) defines a first annular prominence (115) in proximity of said shoulder (113),
**in that** the coupling appendage (22) of said cap (20) has an axial extension, so that the appendage (22) may be inserted inside the container body (11) until it reaches said shoulder (113) with its head end (221), and has a radial extension at the head end (221), so that, once it has reached the shoulder (113), the head end (221) abuts against said shoulder (113), so as to create a first sealing area (T1) between the cap (20) and container body (10),
**in that** the head end portion (221) of the coupling appendage (22) is radially dimensioned such that, when the head end (221) abuts on said shoulder (113), the head end portion (221) interferes with said first annular prominence (115), creating a second sealing zone (T2) between the cap (20) and container body (10),
and **in that** the coupling appendage (22) of said cap (20) comprises a second annular prominence (233) which projects from said lateral coupling surface (23) in proximity of the base (220) of said coupling appendage (22) and is axially positioned so that, when the head end (221) abuts on said shoulder (113), said second annular prominence (222) interferes with the conical guide surface (111), creating a third sealing zone (T3) between the cap (20) and container body (10).

2. The container (1) according to claim 1, wherein said conical guide surface (111) has a conicity between 1/1 and 1/50.

3. The container (1) according to claim 1 or 2, wherein said intermediate surface (112) is conical converging towards the bottom (12).

4. The container (1) according to claim 3, wherein said intermediate surface (112) has a conicity less than the conicity of the conical guide surface (111).

5. The container (1) according to claim 1 or 2, wherein said intermediate surface (112) is cylindrical.

6. The container (1) according to any of the preceding claims, wherein said intermediate surface (112) defines the first annular prominence (115) close to said shoulder (113).

7. The container (1) according to any of the preceding claims, wherein the coupling surface (23) of said coupling appendage (22) is substantially cylindrical.

8. The container (1) according to any of the preceding claims, wherein the head end (221) is counter-shaped to said shoulder (113).

9. The container (1) according to any of the preceding claims, wherein said cap (20) is engageable by screwing onto said container body (10) at said main gripping body (21).

10. The container (1) according to claim 9, wherein said main gripping body (21) is constituted by a cup-shaped body comprising a bottom (210) and a side wall (211), and wherein said coupling appendage (22) extends coaxially inside said cup-shaped body starting from the bottom (210) in such a way that, between the side wall (211) of said cup-shaped body and said coupling appendage, an annular cavity (212) is defined, intended to partially accommodate within it a portion of said container body (10), and wherein the side wall (211) comprises a threaded surface (213) facing towards the inside of said cup-shaped body and intended to engage by screwing a counter-threaded surface (121) made on an outer surface (120) of said container body (10).

11. The container (1) according to any of the claims from 1 to 8, wherein said main gripping body (21) defines at the base (220) of said coupling appendage (22) an abutment ring (214) for the free edge (14) of said container body (10) and wherein said main gripping body (21) is configured to engage said container body (10) only at said abutment ring (214).

12. The container (1) according to any of the preceding claims, wherein said container body (10) is made of a plastic material selected from the group consisting of polypropylene, polyethylene, polystyrene, PET, PVC, polycarbonate, methacrylate and copolymers thereof.

13. The container (1) according to any of the preceding claims, wherein said cap (20) is made of a plastic material selected from the group consisting of polypropylene, polyethylene, polystyrene, PET, PVC, polycarbonate, methacrylate and copolymers thereof.

14. The container (1) according to any of the preceding claims, wherein said container (1) is a jar, in particular for containing biological samples.

15. The container (1) according to any of the claims from 1 to 13, wherein said container (1) is a test tube, in particular for containing biological samples.

## Patentansprüche

1. Hermetisch abdichtbarer Behälter (1) für Flüssigkeiten, umfassend:
- einen Behälterkörper (10), der das Innenvolumen des Behälters lateral bzw. seitlich mittels einer rohrförmigen Behälterwand (11) und unten durch einen Behälterboden (12) begrenzt, von dem sich die rohrförmige Behälterwand (11) erstreckt, wobei der Behälterkörper mit einer Öffnung (13) versehen ist, die durch eine freie Kante bzw. einen freien Rand (14) der rohrförmigen Wand (11) begrenzt ist; und
- eine Kappe (20), die konfiguriert ist, die Öffnung (13) des Behälterkörpers (10) zu verschließen und mit dieser eine hermetische Abdichtung zu bilden,
wobei die Kappe (20) einen Hauptgreif- bzw. -griffkörper (21), der dazu bestimmt ist, außerhalb des Behälterkörpers (10) zu verbleiben, und einen Kopplungsansatz (22) umfasst, der dazu bestimmt ist, durch die Öffnung (13) in das Innere des Behälterkörpers (10) eingesetzt zu werden, sich axial zwischen einer Basis (220) und einem Kopfende (221) erstreckt und eine laterale bzw. seitliche Kopplungsfläche bzw. -oberfläche (23) umfasst, die dazu bestimmt ist, einer Innenfläche bzw. -oberfläche (110) der rohrförmigen Behälterwand (11) zugewandt zu sein,
**dadurch gekennzeichnet, dass** die Innenfläche (110) der rohrförmigen Behälterwand (11) beginnend von der freien Kante (14) in Richtung Boden (12) sequentiell umfasst:
- eine konische Führungsfläche bzw. -oberfläche (111), die in Richtung Boden (12) konvergiert;
- eine Zwischenfläche bzw. -oberfläche (112), welche die konische Führungsfläche (111) mit einer Schulter (113) verbindet;
- eine Endfläche bzw. -oberfläche (114), welche die Schulter (113) mit dem Behälterboden (12) verbindet, wobei die Zwischenfläche (112) einen ersten ringförmigen Vorsprung (115) in der Nähe der Schulter (113) definiert,
dass der Kopplungsansatz (22) der Kappe (20) eine axiale Erstreckung bzw. Verlängerung aufweist, so dass der Ansatz (22) in das Innere des Behälterkörpers (11) eingesetzt werden kann, bis er mit seinem Kopfende (221) die Schulter (113) erreicht, und eine radiale Erstreckung bzw. Verlängerung an dem Kopfende (221) aufweist, so dass das Kopfende (221), sobald es die Schulter (113) erreicht hat, an der Schulter (113) anliegt, um einen ersten Abdichtungsbereich (T1) zwischen der Kappe (20) und dem Behälterkörper (10) zu bilden,
dass der Kopfendabschnitt (221) des Kopplungsansatzes (22) radial derart bemessen ist, dass, wenn das Kopfende (221) an der Schulter (113) anliegt, der Kopfendabschnitt (221) mit dem ersten ringförmigen Vorsprung (115) zusammentrifft, wodurch eine zweite Abdichtungszone (T2) zwischen der Kappe (20) und dem Behälterkörper (10) erzeugt wird,
und dass der Kopplungsansatz (22) der Kappe (20) einen zweiten ringförmigen Vorsprung (233) umfasst, der von der lateralen Kopplungsfläche (23) in der Nähe der Basis (220) des Kopplungsansatzes (22) vorsteht und axial so positioniert ist, dass, wenn das Kopfende (221) an der Schulter (113) anliegt, der zweite ringförmige Vorsprung (222) mit der konischen Führungsfläche (111) zusammentrifft, wodurch eine dritte Abdichtungszone (T3) zwischen der Kappe (20) und dem Behälterkörper (10) erzeugt wird.

2. Behälter (1) nach Anspruch 1, wobei die konische Führungsfläche (111) eine Konizität zwischen 1/1 und 1/50 aufweist.

3. Behälter (1) nach Anspruch 1 oder 2, wobei die Zwischenfläche (112) in Richtung des Bodens (12) konisch konvergierend ist.

4. Behälter (1) nach Anspruch 3, wobei die Zwischenfläche (112) eine Konizität aufweist, die geringer ist als die Konizität der konischen Führungsfläche (111).

5. Behälter (1) nach Anspruch 1 oder 2, wobei die Zwischenfläche (112) zylindrisch ist.

6. Behälter (1) nach einem der vorhergehenden Ansprüche, wobei die Zwischenfläche (112) den ersten ringförmigen Vorsprung (115) nahe der Schulter (113) definiert.

7. Behälter (1) nach einem der vorhergehenden Ansprüche, wobei die Kopplungsfläche (23) des Kopplungsansatzes (22) im Wesentlichen zylindrisch ist.

8. Behälter (1) nach einem der vorhergehenden Ansprüche, wobei das Kopfende (221) gegenläufig zu der Schulter (113) geformt ist.

9. Behälter (1) nach einem der vorhergehenden Ansprüche, wobei die Kappe (20) durch Aufschrauben auf den Behälterkörper (10) an dem Hauptgreifkörper (21) in Eingriff bringbar ist.

10. Behälter (1) nach Anspruch 9, wobei der Hauptgreifkörper (21) aus einem becherförmigen Körper mit einem Boden (210) und einer Seitenwand (211) besteht und wobei sich der Kopplungsansatz (22) koaxial im Inneren des becherförmigen Körpers ausgehend von dem Boden (210) derart erstreckt, dass zwischen der Seitenwand (211) des becherförmigen Körpers und dem Kopplungsansatz ein ringförmiger Hohlraum (212) definiert ist, der dazu bestimmt ist, einen Abschnitt des Behälterkörpers (10) teilweise in sich aufzunehmen, und wobei die Seitenwand (211) eine Gewindefläche bzw. - oberfläche (213) umfasst, die in Richtung Innenseite des becherförmigen Körpers gewandt und dazu bestimmt ist, durch Schrauben in eine Gegengewindefläche bzw. -oberfläche (121) einzugreifen, die an einer Außenfläche bzw. -oberfläche (120) des Behälterkörpers (10) ausgebildet ist.

11. Behälter (1) nach einem der Ansprüche 1 bis 8, wobei der Hauptgreifkörper (21) an der Basis (220) des Kopplungsansatzes (22) einen Anlagering (214) für die freie Kante (14) des Behälterkörpers (10) definiert, und wobei der Hauptgreifkörper (21) konfiguriert ist, nur an dem Anlagering (214) in den Behälterkörper (10) einzugreifen.

12. Behälter (1) nach einem der vorhergehenden Ansprüche, wobei der Behälterkörper (10) aus einem Kunststoffmaterial besteht, das aus der Gruppe bestehend aus Polypropylen, Polyethylen, Polystyrol, PET, PVC, Polycarbonat, Methacrylat und Copolymeren davon ausgewählt ist.

13. Behälter (1) nach einem der vorhergehenden Ansprüche, wobei die Kappe (20) aus einem Kunststoffmaterial besteht, das aus der Gruppe bestehend aus Polypropylen, Polyethylen, Polystyrol, PET, PVC, Polycarbonat, Methacrylat und Copolymeren davon ausgewählt ist.

14. Behälter (1) nach einem der vorhergehenden Ansprüche, wobei der Behälter (1) ein Gefäß ist, insbesondere zur Aufnahme bzw. zum Enthalten biologischer Proben.

15. Behälter (1) nach einem der Ansprüche 1 bis 13, wobei der Behälter (1) ein Reagenzglas ist, insbesondere zur Aufnahme bzw. zum Enthalten biologischer Proben.

## Revendications

1. Contenant (1) hermétiquement scellable pour liquides, comprenant :
- un corps de contenant (10) délimitant le volume intérieur du contenant latéralement par une paroi de contenant tubulaire (11) et en-dessous par un fond de contenant (12) à partir duquel ladite paroi de contenant tubulaire (11) s'étend, ledit corps de contenant étant pourvu d'une bouche (13) délimitée par un bord libre (14) de ladite paroi tubulaire (11) ; et
- un couvercle (20) configuré pour fermer la bouche (13) du corps de contenant (10) et constituer une fermeture hermétique avec celle-ci,
dans lequel le couvercle (20) comprend un corps saisissant principal (21) qui est destiné à rester à l'extérieur dudit corps de contenant (10), et un appendice de liaison (22), qui est destiné à être inséré à l'intérieur dudit corps de contenant (10) par ladite bouche (13), s'étend axialement entre une base (220) et une extrémité de tête (221), et comprend une surface latérale de liaison (23) destinée à être en regard d'une surface intérieure (110) de ladite paroi de contenant tubulaire (11),
**caractérisé en ce que** la surface intérieure (110) de ladite paroi de contenant tubulaire (11) comprend, dans l'ordre, à partir du bord libre (14) vers le fond (12) :
- une surface de guidage (111) conique convergeant vers le fond (12) ;
- une surface intermédiaire (112) reliant ladite surface de guidage (111) conique à un épaulement (113) ;
- une surface d'extrémité (114) reliant ledit épaulement (113) au fond de contenant (12), dans lequel ladite surface intermédiaire (112) définit une première proéminence (115) annulaire à proximité dudit épaulement (113),
**en ce que** l'appendice de liaison (22) dudit couvercle (20) a une étendue axiale, de façon que l'appendice (22) puisse être inséré à l'intérieur du corps de contenant (11) jusqu'à ce qu'il arrive à l'épaulement (113) avec son extrémité de tête (221), et a une étendue radiale à l'extrémité de tête (221) de façon que, une fois arrivé à l'épaulement (113), l'extrémité de tête (221) bute sur ledit épaulement (113) afin de créer une première zone de scellage (T1) entre le couvercle (20) et le corps de contenant (10),
**en ce que** la partie d'extrémité de tête (221) de l'appendice de liaison (22) a des dimensions radiales telles que, lorsque l'extrémité de tête (221) bute sur ledit épaulement (113), la partie d'extrémité de tête (221) vienne en contact avec ladite première proéminence (115) annulaire, créant une deuxième zone de scellage (T2) entre le couvercle (20) et le corps de contenant (10),
et **en ce que** l'appendice de liaison (22) dudit couvercle (20) comprend une deuxième proéminence (233) annulaire qui s'étend à partir de ladite surface latérale de liaison (23) à proximité de la base (220) dudit appendice de liaison (22) et est positionnée axialement de façon que, lorsque l'extrémité de tête (221) bute sur ledit épaulement (113), ladite deuxième proéminence (222) annulaire vienne en contact avec la surface de guidage (111) conique, créant une troisième zone de scellage (T3) entre le couvercle (20) et le corps de contenant (10).

2. Contenant (1) selon la revendication 1, dans lequel ladite surface de guidage (111) conique a une conicité entre 1/1 et 1/50.

3. Contenant (1) selon la revendication 1 ou 2, dans lequel ladite surface intermédiaire (112) est conique convergeant vers le fond (12).

4. Contenant (1) selon la revendication 3, dans lequel ladite surface intermédiaire (112) a une conicité inférieure à la conicité de la surface de guidage (111) conique.

5. Contenant (1) selon la revendication 1 ou 2, dans lequel ladite surface intermédiaire (112) est cylindrique.

6. Contenant (1) selon l'une des revendications précédentes, dans lequel ladite surface intermédiaire (112) définit la première proéminence (115) annulaire proche dudit épaulement (113).

7. Contenant (1) selon l'une des revendications précédentes, dans lequel la surface de liaison (23) dudit appendice de liaison (22) est sensiblement cylindrique.

8. Contenant (1) selon l'une des revendications précédentes, dans lequel l'extrémité de tête (221) a une forme complémentaire à celle dudit épaulement (113).

9. Contenant (1) selon l'une des revendications précédentes, dans lequel le couvercle (20) peut être fixé en le vissant sur ledit corps de contenant (10) audit corps saisissant principal (21).

10. Contenant (1) selon la revendication 9, dans lequel ledit corps saisissant principal (21) est constitué par un corps en forme de coupe comprenant un fond (210) et une paroi latérale (211), et dans lequel ledit appendice de liaison (22) s'étend coaxialement à l'intérieur dudit corps en forme de coupe à partir du fond (210) de façon telle que, entre la paroi latérale (211) dudit corps en forme de coupe et ledit appendice de liaison, une cavité annulaire (212) soit définie, destinée à loger partiellement en son intérieur une partie dudit corps de contenant (10), et dans lequel la paroi latérale (211) comprend une surface filetée (213) orientée vers l'intérieur dudit corps en forme de coupe et destinée à être fixée, en la vissant, sur une surface (121) à contre-filetage réalisée sur une surface extérieure (120) dudit corps de contenant (10).

11. Contenant (1) selon l'une des revendications là 8, dans lequel ledit corps saisissant principal (21) définit à la base (220) dudit appendice de saisie (22) un anneau de butée (214) pour le bord libre (14) dudit corps de contenant (10) et dans lequel le corps saisissant principal (21) est configuré pour être fixé sur le corps de contenant (10) uniquement audit anneau de butée (214).

12. Contenant (1) selon l'une des revendications précédentes, dans lequel le corps de contenant (10) est fait en une matière plastique choisie dans le groupe constitué de polypropylène, polyéthylène, polystyrène, PET, PVC, polycarbonate, méthacrylate et copolymères de ceux-ci.

13. Contenant (1) selon l'une des revendications précédentes, dans lequel ladite coupe (20) est faite en une matière plastique choisie dans le groupe constitué de polypropylène, polyéthylène, polystyrène, PET, PVC, polycarbonate, méthacrylate et copolymères de ceux-ci.

14. Contenant (1) selon l'une des revendications précédentes, dans lequel ledit contenant (1) est un pot, notamment pour contenir des échantillons biologiques.

15. Contenant (1) selon l'une des revendications 1 à 13, dans lequel ledit contenant (1) est un tube de test, notamment pour contenir des échantillons biologiques.
